# EUROPEAN PATENT APPLICATION

(11) **EP 3 929 937 A1**
(43) Date of publication of application: **29.12.2021**
(21) Application number: 20181463.9
(22) Date of filing: 22.06.2020
(51) Int. Cl.: G16H 40/20, G16H 50/20

(54) **DETERMINING THE IMPACT OF COVID-19 PROGRESSION**

(71) Applicant: Bayer Aktiengesellschaft, 51373 Leverkusen (DE)
(72) Inventor: RAMOS DOS SANTOS, Thiago, IG1 2ZD Ilford (GB); BONACCORSO, Guiseppe, 10585 Berlin (DE); NTHEI MULLI, Justus Jakob, 113355 Berlin (DE); JAPP, Seda Sada, 10439 Berlin (DE); HOOGE, Jens, 13088 Berlin (DE); PURTORAB, Marvin, RG2 6AD Reading (GB); LORIO, Sara, RG4 5DQ Reading (GB)
(74) Representative: BIP Patents

(57) **Abstract**

The invention relates to computer-implemented method for determining the impact of COVID-19 progression of a subject patient suffering from COVID-19 on a hospital and its Intensive Care Unit (ICU), comprising: receiving patient data of a subject patient who has been infected with SARS-CoV-2 and hospitalized, wherein the patient data comprises at least one image of the lung of the subject patient; inputting the patient data into a prediction model trained via machine learning with a set of training data, wherein the prediction model is trained by retrospective data to predict the relationship between visible features of COVID-19 in the lung and at least one characteristic of the COVID-19 progression of the subject patient suffering from COVID-19; receiving, as an output from the prediction model, at least one characteristic of the COVID-19 disease progression of the subject patient suffering from COVID-19.

## Description

### FIELD OF THE INVENTION

The present invention relates to a method for determining the impact of a COVID-19 progression of a subject patient suffering from COVID-19 on a hospital and its Intensive Care Units (ICUs). The present invention provides methods, systems and computer-readable storage media for determining the impact of the COVID-19 progression of a subject patient suffering from COVID-19 on a hospital and its ICUs and furthermore for determining the treatment capacity for a plurality of subject patients suffering from COVID-19 in a hospital.

### BACKGROUND OF THE INVENTION

Coronavirus disease 2019 (COVID-19) is caused by SARS-CoV-2, a newly emergent coronavirus, that was first recognized in Wuhan, China, in December 2019. Genetic sequencing of the virus suggests that it is a betacoronavirus closely linked to the SARS virus. By way of definition, a symptomatic COVID-19 case is a person who has developed signs and symptoms suggestive of COVID-19.

While most people with COVID-19 develop only mild (40%) or moderate (40%) symptoms, approximately 15% suffer from severe symptoms and require oxygen support, and 5% reach a critical state with complications such as respiratory failure, acute respiratory distress syndrome (ARDS), sepsis and septic shock, thromboembolism, and/or multiorgan failure, including acute kidney injury and cardiac injury. Older age, smoking and underlying noncommunicable diseases (NCDs), such as diabetes, hypertension, cardiac disease, chronic lung disease and cancer, have been reported as risk factors for severe disease and death (Clinical Management of COVID-19, Interim Guidance, May 27, 2020, WHO, WHO reference number: WHO/2019-nCoV/clinical/2020. 5).

Governments, international agencies and health systems have an obligation to ensure, to the best of their ability, adequate provision of health care for all. However, this may be difficult during a pandemic, when health resources are likely to be limited. Setting priorities and rationing resources in this context means making tragic decisions. Such decisions may include access to hospitals, intensive care units (ICUs), ventilators, vaccines and medicines. This resource allocation problem is also referred to as an ethical triage (see also Ethics and COVID-19: resource allocation and priority-setting, WHO, WHO reference number: WHO/RFH/20.2.). There is a need to address this problem in the context of the COVID-19 pandemic.

### SUMMARY OF THE INVENTION

It would be advantageous to have a way to mitigate the resource allocation and priority-setting problems associated with the COVID-19 pandemic. In particular, there is a need to manage resources in a hospital in order to ensure the best treatment and recovery for all those subject patients that suffer from COVID-19 and that have been hospitalized. Overall, there is an aim to strengthen clinical frontline management in general.

The object of the present invention is solved with the subject matter of the independent claims, wherein further embodiments are incorporated in the dependent claims.

A first aspect of the invention provides a computer-implemented method for determining the impact of COVID-19 progression of a subject patient suffering from COVID-19 on a hospital and its Intensive Care Unit (ICU), comprising:
- receiving patient data of a subject patient who has been infected with SARS-CoV-2 and hospitalized, wherein the patient data comprises at least one image of the lung of the subject patient,
- inputting the patient data into a prediction model trained via machine learning with a set of training data, wherein the prediction model is trained by retrospective data to predict the relationship between visible features of COVID-19 in the lung and at least one characteristic of the COVTD-19 progression of the subject patient suffering from COVID-19,
- receiving, as an output from the prediction model, at least one characteristic of the COVID-19 disease progression of the subject patient suffering from COVID-19.

In other words, at least one image of the lung of a subject patient suffering from COVID-19 is acquired at the beginning of the hospitalization. With the method described herein, the medical personnel in the hospital where the subject patient has been hospitalized can very quickly identify the resources that are needed to treat the subject patient while he/she is in the hospital. The method provides important information about the impact on the hospital and ICUs which depends on the disease progression of an individual subject patient suffering from COVID-19. This analysis provides decisive information to allocate resources and set priorities as early as possible in the hospital. In summary, the method facilitates professional health-care management of COVID-19 patients in a hospital and ensures the best treatment and recovery for all patients that are suffering from the disease.

In an example, at least one characteristic of the COVID-19 progression is selected from the group of:
- Need for ventilation of the subject patient suffering from COVID-19,
- Time until subject patient suffering from COVID-19 needs ventilation if a need for ventilation has been determined,
- Length of stay of the subject patient suffering from COVID-19 with ventilation if a need for ventilation has been determined,
- Need for the subject patient suffering from COVID-19 for intensive care in an ICU of a hospital,
- Time until subject patient suffering from COVID-19 needs intensive care in an ICU of a hospital,
- Length of stay of the subject patient suffering from COVID-19 in the ICU of a hospital,
- Length of stay of the subject patient suffering from COVID-19 in the hospital.

In this manner, the resources for ventilation, ICUs and the length of stay in a hospital can be assessed for each individual subject patient suffering from COVID-19 as early as possible (e.g., the first day when the subject patient is hospitalized). The hospital can determine the resource allocations which are likely to be necessary for the individual patient. (e.g., for a future time period of 10 to 15 days as the method described herein enables the determination of the COVID-19 progression).

In an example, the patient data further comprise at least one image of the aorta and/or heart of the subject patient suffering from COVID-19, and wherein the prediction model is trained by retrospective data to predict the relationship between visible features of COVID-19 in the lung in addition to visible features of COVID-19 in the aorta and/or heart and at least one characteristic of the COVID-19 disease progression of the subject patient suffering from COVID-19.

Thus, in order to improve the prediction model further patient data can be taken into consideration. Image data of the aorta and/or heart is useful to enhance the prediction model as described herein.

In an example, the visible features of COVID-19 in the lung comprise pulmonary abnormalities such as fibrosis and/or emphysema and wherein the visible features of COVID-19 in the heart and/or aorta comprise perfusion abnormalities.

In an example, the patient data comprises at least two images of the heart and/or aorta for at least two different time points.

Thus, protocols of the evaluation of myocardial perfusion require images like Computed Tomography (CT) for at least two different time points. Ideally, one time point of the image acquisition is chosen during rest (baseline) and another time point is chosen during stress (hyperemia) conditions of the patient.

In an example, the patient data comprises of at least one image selected from the group of a CT image, and an x-ray image.

In an example, an output of the prediction model is at least one characteristic of the COVID-10 progression of a subject patient and its respective probability of occurrence.

A second aspect of the invention relates to a computer-implemented method for the determination of the treatment capacity for a plurality of subject patients suffering from COVID-19 in a hospital comprising:
- analyzing at least one characteristic of a COVID-19 progression from a plurality of subject patients suffering from COVID-19 and hospitalized as determined with a method according to the first aspect of the invention,
- determining the treatment capacity for a plurality of subject patients suffering from COVID-19 in the hospital as a function of the analysis of the at least one characteristic of the COVID-19 progression of the plurality of subject patients suffering from COVID-19,
- receiving, as an output, the determination of the treatment capacity for a plurality of subject patients suffering from COVID-19 in the hospital.

In other words, the analysis of the outcome of the prediction model of all COVID-19 patients that are hospitalized, and the comparison of the data, enables the determination of the required resources for a certain time period. (e.g., for the next 10-15 days). As hospitals have a certain limited number of ventilation devices, ICUs and beds available for COVID-19 subject patients, the described method can now determine the treatment capacity within a certain time period in the future. If the number of COVID-19 subject patients which require ventilation and/or ICU exceeds the treatment capacity in the hospital within the time period, the medicinal personnel in the hospital can take measures to prevent resource allocation problems and the ethical triage by requesting further ventilation devices from a third party and/or transferring COVID-19 subject patients to other hospitals prior to when the symptoms of COVID-19 become so severe that a subject patient transfer is not possible anymore for example.

In an example, the at least one characteristic of a COVID-19 progression comprises at least one of the following:
- Need for ventilation of the plurality of subject patients suffering from COVID-19,
- Time until the plurality of subject patients suffering from COVID-19 needs ventilation if need for ventilation has been determined,
- Length of stay of the plurality of subject patients suffering from COVID-19 with ventilation if a need for ventilation has been determined,
- Need for the plurality of subject patients suffering from COVID-19 for intensive care in an ICU of the hospital,
- Time until the plurality of subject patients suffering from COVID-19 need intensive care in an ICU of the hospital,
- Length of stay of the plurality of subject patients suffering from COVID-19 in the ICU of the hospital,
- Length of stay of the plurality of subject patients suffering from COVID-19 in the hospital.

In an example, the determination of the treatment capacity for a plurality of subject patients suffering from COVID-19 in a hospital as a function of the analysis of the at least one characteristic of the COVID-19 progression of the plurality of subject patients suffering from COVID-19 comprises at least one of the following:
- allocation of available ventilation devices in the hospital to a plurality of subject patients suffering from COVID-19 according to the determined characteristics of the COVID-19 progression for each subject patient,
- allocation of ICUs in the hospital to a plurality of subject patients suffering from COVID-19 according to the determined characteristics of the COVID-19 progression for each subject patient.

In an example, the treatment capacity for a plurality of subject patients suffering from COVID-19 in a hospital as a function of the analysis of the at least one characteristic of the COVID-10 progression of the plurality of subject patients suffering from COVID-19 comprises at least one of the following:
- Request of ventilation devices from a third party if the need for ventilation devices has been predicted to succeed the availability of ventilation devices in the hospital,
- Transport of the required ventilation devices from the third party to the hospital,
- Request the hospitalization of (an) eligible subject patient(s) suffering from COVID-19 in another hospital with available ICUs if the need for ICUs in the hospital has been predicted to exceed the availability of ICUs in the hospital,
- Transfer of (a) eligible subject patient(s) suffering from COVID-19 to another hospital with available ICUs.

Thus, the outcome of the treatment capacity analysis are anticipatory measures at a point in time when the ethical triage problem not yet arises. Therefore, the use of anticipatory measures could avoid ethical triage issues.

A third aspect of the invention relates to a system comprising:
- a patient data receiving unit for receiving patient data of a subject patient who has been infected with SARS-CoV-2, the patient data comprising at least one image of the lung of the subject patient;
- a memory;
- a processing unit in communication with the memory and configured with processor-executable instructions to perform operations comprising:
- feeding the patient data of the subject patient into a prediction model, wherein the prediction model is trained by retrospective data to predict the relationship between visible features of COVID-19 in the lung and at least one characteristic of the COVID-19 progression of the subject patient suffering from COVID-19,
- receiving as an output from the prediction model the at least one characteristic of the COVID-19 progression of the subject patient suffering from COVID-19,
- outputting the at least one characteristic of the COVID-19 progressoin of the subject patient by an output unit.

A fourth aspect of the invention relates to a system comprising:
- a patient data receiving unit for receiving patient data of a plurality of subject patients who have been infected with SARS-CoV-2, the patient data comprising at least one image of the lung of the plurality of subject patients;
- a memory;
- a processing unit in communication with the memory and configured with processor-executable instructions to perform operations comprising:
- feeding the plurality of patient data from a plurality of subject patients into a prediction model, wherein the prediction model is trained by retrospective data to predict the relationship between visible features of COVID-19 in the lung and at least one characteristic of the COVID-19 progression of a subject patient suffering from COVID-19,
- receiving as an output from the prediction model at least one characteristic of the COVID-19 progression of a plurality of subject patients suffering from COVID-19,
- analyzing at least one characteristic of the COVID-19 progression of the plurality of subject patients suffering from COVID-19,
- determining the treatment capacity for a plurality of subject patients suffering from COVID-19 in a hospital as a function of the comparison of the at least one characteristic of the COVID-19 progression of the plurality of subject patients suffering from COVID-19,
- outputting the results of the determination of the treatment capacity in a hospital for a plurality of subject patients suffering from COVID-19 progression by an output unit.

According to a fifth aspect of the invention, there is provided a non-transitory computer-readable storage medium comprising processor-executable instructions with which to perform an operation for determining the impact of the COVID-19 progression of a subject patient suffering from COVID-19 on a hospital and its ICUs, the operation comprising:
- receiving patient data of a subject patient who has been infected with SARS-CoV-2, the patient data comprising at least one image of the lung of the subject patient,
- inputting the patient data into a prediction model trained via machine learning with a set of training data, wherein the prediction model is trained by retrospective data to predict the relationship between visible features of COVID-19 in the lung and at least one characteristic of the COVID-19 progression of the subject patient suffering from COVID-19,
- receiving as an output from the prediction model the at least one characteristic of the COVID-19 progression of the subject patient suffering from COVID-19.

A sixth aspect of the invention relates to a non-transitory computer-readable storage medium comprising processor-executable instructions with which to perform an operation for determining the treatment capacity for a plurality of subject patients suffering from COVID-19 in a hospital, the operation comprising:
- analyzing at least one characteristic of the of a COVID-19 progression from a plurality of subject patients suffering from COVID-19 and hospitalized as determined with the operations as described according to the fifth aspect of the invention,
- determining the treatment capacity for a plurality of subject patients suffering from COVID-19 in the hospital as a function of the comparison of the at least one characteristic of the COVID-19 progression of the plurality of subject patients suffering from COVID-19,
- receiving as an output the results of the determination of the treatment capacity for a plurality of subject patients suffering from COVID-19 in the hospital.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic example of the computer-implemented method (100) for determining the impact of COVID-19 progression of a subject patient suffering from COVID-19 on a hospital and its ICUs.
Fig. 2 illustrates schematically an exemplary convolutional neural network (CNN) which can be used to determine the impact of the COVID-19 disease progression of a subject patient suffering from COVID-19 on a hospital and its ICUs.
Fig. 3 illustrates an exemplary recurrent neural network (RNN).
Fig. 4 illustrates an exemplary training and deployment of a neural network.
Fig. 5 shows an exemplary artificial neuronal network with three subnetworks.
Fig. 6 shows a schematic example of the computer-implemented method (200) for determining the treatment capacity for a plurality of subject patients suffering from COVID-19 in a hospital.

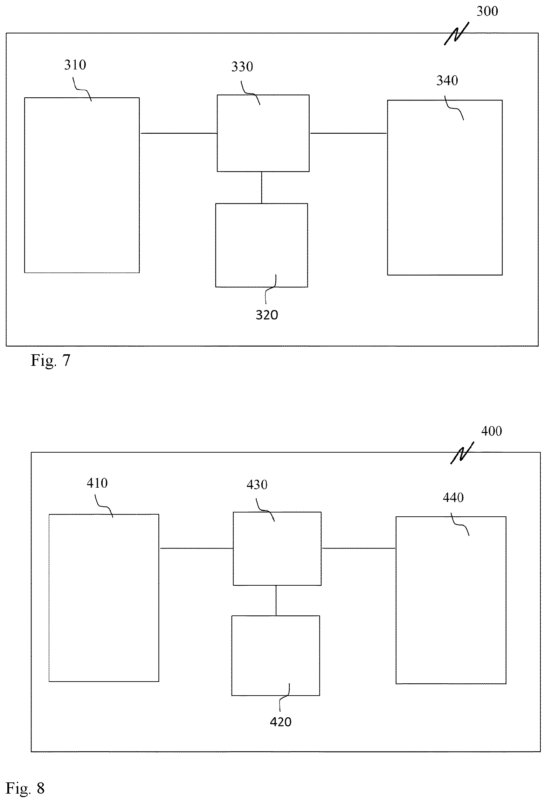

Fig. 7 shows a schematic example of the system (300) of the invention.
Fig. 8 shows a schematic example of the system (400) of the invention.

### DETAILED DESCRIPTION OF EMBODIMENTS

The invention relates in a first embodiment to a computer-implemented method 100 for determining the impact of the COVID-19 progression of a subject patient suffering from COVID-19 on a hospital and its ICUs comprising:
- 110; receiving patient data of a subject patient who has been infected with SARS-CoV-2 and hospitalized, wherein the patient data comprises at least one image of the lung of the subject patient,
- 120; inputting the patient data into a prediction model trained via machine learning with a set of training data, wherein the prediction model is trained by retrospective data to predict the relationship between visible features of COVID-19 in the lung and at least one characteristic of the COVID-19 progression of the subject patient suffering from COVID-19,
- 130; receiving, as an output from the prediction model, at least one characteristic of the COVID-19 disease progression of the subject patient suffering from COVID-19.

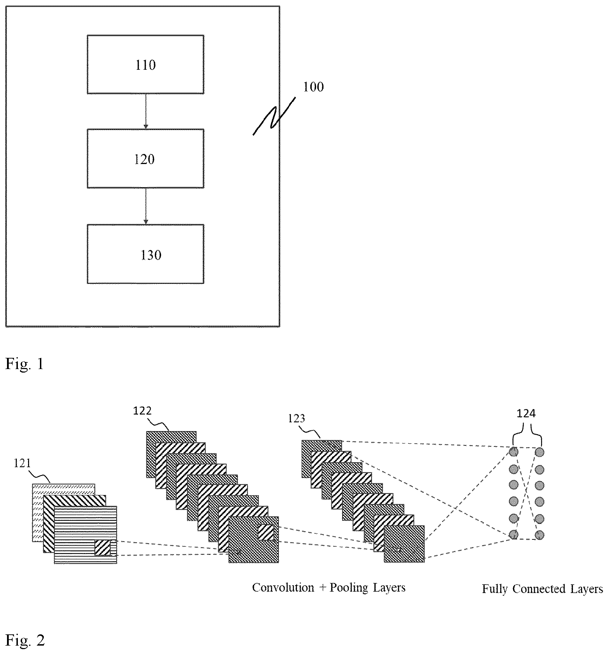

Fig. 1 shows the method 100 schematically in the form of a flow chart.

In an example, the determination of the impact of the COVID-19 progression of a subject patient suffering from COVID-19 on a hospital and its ICUs relates to the determination of the resources that are required in the hospital with its ICUs to treat the subject patient (for example until the subject patient can be discharged from the hospital).

In an example, the method comprises the additional step of: determining the impact on the resources of the hospital and its ICUs as a function of the output of the prediction model.

In an example, the "resources" of the hospital and its ICUs are the availability for ventilation devices and/or ICUs in the hospital, preferably anticipatory for a certain time period (such as e.g. a view days) in the future as further discussed herein.

In an example, the subject patient who has been infected with SARS-CoV-2 has been tested positive for the virus, preferably with laboratory confirmation.

In an example, RT-PCR diagnostics is/has been used to test a subject patient suspected to have contracted COVID-19.

In an example, the subject patient who has been infected with SARS-CoV-2 has been hospitalized and the patient data is acquired and analyzed with the method described herein at the same day when the subject patient has been hospitalized.

In an example, the method as described is performed within a time period of 24 to 48, preferably within a time period of 24 hours when the subject patient is hospitalized.

In an example, the method as described is performed by medicinal personnel at the hospital where the subject patient who has been infected with SARS-CoV-2 has been hospitalized.

In an example, the prediction model is configured to determine from the patient data one or more characteristics of the COVID-19 progression of the subject patient suffering from COVID-19. Preferably, the prediction model was trained with a plurality of images of the lung of a plurality of patients that have suffered from COVID-19 and for which each patient the characteristics of the COVID-19 progression has been determined in the past. The aim of the training is to create a regression function which relates the visual features of the images of the lung of a subject patient who has been infected with SARS-CoV-2 with at least one characteristic of the COVID-19 progression.

In an example, the prediction model can be an artificial neural network. The present invention serves in particular for determining at least one characteristic of the COVID-19 progression of a subject patient suffering from COVID-19 by means of an artificial neural network which is based on a learning process referred to as backpropagation.

Backpropagation is understood herein as a generic term for a supervised learning process with error feedback. There are a variety of backpropagation algorithm e.g. Quickprop, Resilient Propagation (RPROP). Details of setting up an artificial neural network and training the network can be found e.g. in C. C. Aggarwal: Neural Networks and Deep Learning, Springer 2018, ISBN 978-3-319-94462-3.

In an example, the present invention preferably uses an artificial neural network comprising at least three layers of processing elements: a first layer with input neurons (nodes), an *N^{th}* layer with at least one output neuron (node), and *N-2* inner layers, where *N* is a natural number greater than 2. Preferably N is equal to 3 or greater than 3. In such a network, the output neuron(s) serve(s) to predict at least one characteristic of the COVID-19 progression of a subject patient suffering from COVID-19. The input neurons serve to receive input values (the sequence of images or data derived therefrom). The processing elements are interconnected in a predetermined pattern with predetermined connection weights therebetween. The network has been previously trained to simulate at least one characteristic of the COVID-19 progression of a subject patient suffering from COVTD-19 as a function of the visual features of at least one image of the lung of the subject patient. When trained, the connection weights between the processing elements contain information regarding the correlation between the visual features of the lung image (input) of the subject patient and the at least one characteristic of the COVID-19 progression (output) of the subject patient, which can be used to predict at least one characteristic of the COVID-19 progression based on at least one lung image of a new patient who has been infected with SARS-CoV-2.

In an example, a network structure can be constructed with input nodes for each input variable, one or more hidden nodes and at least one output node for the parameter representing the at least one characteristic of the COVID-19 progression. The nodes are connected by weighted connections between input and hidden, between hidden and hidden (in case N > 3), and between hidden and output node(s). Additional threshold weights are applied to the hidden and output nodes. Each network node represents a simple calculation of the weighted sum of inputs from prior nodes and a non-linear output function. The combined calculation of the network nodes relates the inputs to the output(s). Separate networks can be developed for each property measurement or groups of properties can be included in a single network. Training estimates network weights that allow the network to calculate (an) output value(s) close to the measured (i.e., test) output value(s). A supervised training method can be used, in which the output data is used to direct the training of the network weights. The network weights are initialized with small random values or with the weights of a prior partially trained network. The process data inputs are applied to the network and the output values are calculated for each training sample. The network output values are compared to the measured output values. A backpropagation algorithm is applied to correct the weight values in directions that reduce the error between measured and calculated outputs. The process is iterated until no further error reduction can be achieved. A cross-validation method is employed to split the data into training and testing data sets. The training data set is used in the backpropagation training of the network weights. The testing data set is used to verify that the trained network generalizes to make good predictions. The best network weight set can be taken as the one that best predicts the outputs of the test data set. Similarly, varying the number of hidden nodes and determining the network that performs best with the test data optimizes the number of hidden nodes. Forward prediction uses the trained network to calculate estimates of property outputs for at least one characteristic of the COVID-19 progression. At least one image of the lung of a COVID-19 subject patient is input to the trained network. A feed forward calculation through the network is made to predict the output property value(s). The predicted measurements can be compared to (a) property target value(s) or tolerance(s). Since the method of the invention is based on historical data of property values, the prediction of property values using such method typically have an error approaching the error of the empirical data, so that the invention predictions are often just as accurate as verification experiments.

In an example, the prediction model is or comprises a Convolutional Neural Network (CNN). A CNN is a class of deep neural networks, most commonly applied to image analysis. A CNN comprises an input layer with input neurons, an output layer with at least one output neuron, as well as multiple hidden layers between the input layer and the output layer. The hidden layers of a CNN typically consist of convolutional layers, ReLU (Rectified Linear Units) layer (i.e., activation function), pooling layers, fully connected layers and normalization layers. The nodes in the CNN input layer are organized into a set of "filters" (feature detectors), and the output of each set of filters is propagated to nodes in successive layers of the network. The computations for a CNN include applying the convolution mathematical operation (cross-correlation operation) to each filter to produce the output of that filter. Convolution is a specialized kind of mathematical operation performed by two functions to produce a third function that is a modified version of one of the two original functions. In convolutional network terminology, the first function to the convolution can be referred to as the input, while the second function can be referred to as the convolution kernel. The output may be referred to as the feature map. For example, the input to a convolution layer can be a multidimensional array of data that defines the various color components of an input image. The convolution kernel can be a multidimensional array of parameters, where the parameters are adapted by the training process for the neural network. When moving from image to video analysis with CNNs, the complexity of the task is increased by the extension into the temporal dimension. This dimension can be processed by introducing 3D convolutions, additional multi-frame optical flow images, or recurrent neural networks (RNNs). RNN are a family of feedforward neural networks that include feedback connections between layers. RNNs enable modeling of sequential data by sharing parameter data across different parts of the neural network. The architecture for an RNN includes cycles. The cycles represent the influence of a present value of a variable on its own value at a future time, as at least a portion of the output data from the RNN is used as feedback for processing subsequent input in a sequence. When analyzing video material (a sequence of images), space and time can be treated as equivalent dimensions and processed via 3D convolutions. This was explored in the works of Baccouche et al. (Sequential Deep Learning for Human Action Recognition; International Workshop on Human Behavior Understanding, Springer 2011, pages 29-39) and Ji et al. (3D Convolutional Neural Networks for Human Action Recognition, IEEE transactions on pattern analysis and machine intelligence, 35(1), 221-231). On the other hand, one can train different networks, responsible for time and space, and finally fuse the features, which can be found in publications of Karpathy et al. (Large-scale Video Classification with Convolutional Neural Networks; Proceedings of the IEEE conference on Computer Vision and Pattern Recognition, 2014, pages 1725-1732), and Simonyan & Zisserman (Two-stream Convolutional Networks for Action Recognition in Videos; Advances in neural information processing systems, 2014, pages 568-576).

Fig. 2 illustrates schematically an exemplary CNN which can be used to determine the impact of the COVID-19 disease progression of a subject patient suffering from COVID-19 on a hospital and its ICUs. Fig. 2 illustrates various layers within a CNN. As shown in Fig. 2, an exemplary CNN used to identify within patient data one or more characteristic of the COVID-19 progression can receive input 121 describing the red, green, and blue (RGB) components of an input lung image. The input 121 can be processed by multiple convolutional layers (e.g., convolutional layer and pooling layers 122, 123). The output from the multiple convolutional layers may optionally be processed by a set of fully connected layers 124. Neurons in a fully connected layer have full connections to all activations in the previous layer. The output from the fully connected layers 124 can be used to generate an output result from the network. The output result can be the determination of at least one characteristic of the COVID-19 progression from a patient subject suffering from COVID-19. The activations within the fully connected layers 124 can be computed using matrix multiplication instead of convolution. Not all CNN implementations make use of fully connected layers. For example, in some implementations, the convolutional layer and pooling layer 123 can generate output for the CNN. The convolutional layers are sparsely connected, which differs from traditional neural network configuration found in the fully connected layers 124. Traditional neural network layers are fully connected, such that every output unit interacts with every input unit. However, the convolutional layers are sparsely connected because the output of the convolution of a field is input (instead of the respective state value of each of the nodes in the field) to the nodes of the subsequent layer, as illustrated. The kernels associated with the convolutional layers perform convolution operations, the output of which is sent to the next layer. The dimensionality reduction performed within the convolutional layers is one aspect that enables the CNN to process large images.

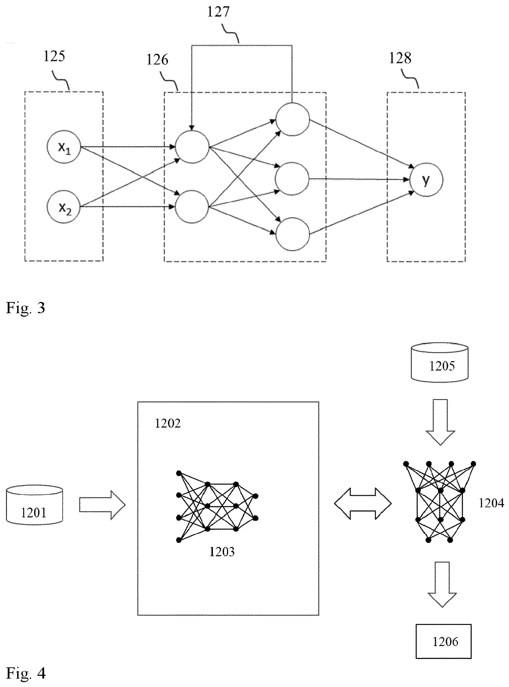

Fig. 3 illustrates an exemplary RNN. In a RNN, the previous state of the network influences the output of the current state of the network. RNNs can be built in a variety of ways using a variety of functions. The use of RNNs generally revolves around using mathematical models to predict the future based on a prior sequence of inputs. The illustrated RNN can be described has having an input layer 125 that receives an input vector, hidden layers 126 to implement a recurrent function, a feedback mechanism 127 to enable a 'memory' of previous states, and an output layer 128 to output a result. The RNN operates based on time-steps. The state of the RNN at a given time step is influenced based on the previous time step via the feedback mechanism 127. For a given time step, the state of the hidden layers 126 is defined by the previous state and the input at the current time step. An initial input (x₁) at a first-time step can be processed by the hidden layer 126. A second input (x₂) can be processed by the hidden layer 126 using state information that is determined during the processing of the initial input (x₁). A given state can be computed as *sₜ =f(Uxₜ* + *Wsₜ₋₁),* where *U* and *W* are parameter matrices. The function *f* is generally a nonlinearity, such as the hyperbolic tangent function (tanh) or a variant of the rectifier function *f(x)* = *max(0, x).* However, the specific mathematical function used in the hidden layers 126 can vary depending on the specific implementation details of the RNN

Fig. 4 illustrates an exemplary training and deployment of a neural network. Once a given network has been structured for a task the neural network is trained using a training dataset 1201. To start the training process the initial weights may be chosen randomly or by pre-training using a deep belief network. The training cycle then be performed in either a supervised or unsupervised manner. Supervised learning is a learning method in which training is performed as a mediated operation, such as when the training dataset 1201 includes input paired with the desired output for the input, or where the training dataset includes input having known output and the output of the neural network is manually graded. The network processes the inputs and compares the resulting outputs against a set of expected or desired outputs. Errors are then propagated back through the system. The training framework 1202 can adjust the weights that control the untrained neural network 1203. The training framework 1202 can provide tools to monitor how well the untrained neural network 1203 is converging towards a model suitable to generating correct answers based on known input data. The training process occurs repeatedly as the weights of the network are adjusted to refine the output generated by the neural network. The training process can continue until the neural network reaches a statistically desired accuracy associated with a trained neural network 1204. The trained neural network 1204 can then be deployed to implement any number of machine learning operations. Patient data 1205 can be inputted into the trained neural network 1204 to determine at least one characteristic of the COVID-19 progression of the subject patient suffering from COVID-19 1206.

According to an example, the at least one characteristic of the COVID-19 progression is selected from the group of:
- Need for ventilation of the subject patient suffering from COVID-19,
- Time until subject patient suffering from COVID-19 needs ventilation if a need for ventilation has been determined,
- Length of stay of the subject patient suffering from COVID-19 with ventilation if a need for ventilation has been determined,
- Need for the subject patient suffering from COVID-19 for intensive care in an ICU of a hospital,
- Time until subject patient suffering from COVID-19 needs intensive care in an ICU of a hospital,
- Length of stay of the subject patient suffering from COVID-19 in the ICU of a hospital,
- Length of stay of the subject patient suffering from COVID-19 in the hospital.

In an example, the "time until ventilation and/or ICU is needed" as well as "the length of stay with ventilation/in the ICU) is indicated in hours or days.

In an example, subject patient who has been infected with SARS-CoV-2 where at least one characteristic of the COVID-19 progression as determined with the method discussed herein is that the degree of impact of the disease progression will be minor, may present to an emergency unit, primary care/outpatient department, or be encountered during community outreach activities, such as home visits or by telemedicine. For such subject patients no need for ventilation or ICU has been determined. Also, patients with moderate illness may not require emergency interventions or hospitalization.

In an example, subject patient who has been infected with SARS-CoV-2 where at least one characteristic of the COVID-19 progression as determined with the method discussed herein is that the degree of impact of the disease progression will be major may need ventilation and/or ICU in the near future.

In an example, for those subject patients where it is determined that the impact of the disease progression will be major, a need of ventilation and/or a need of intensive care in an ICU can be determined. For such cases the method described herein determines the time until the subject patient needs ventilation and/or ICU. In addition, the length of stay of the subject patient with ventilation support and/or length of stay in an ICU will be determined.

In an example, ventilation refers to the pulse oximeters, functioning oxygen systems and disposable, single-use, oxygen-delivering interfaces (nasal cannula, Venturi mask, and mask with reservoir bag).

It is recommended to ventilate subject patients with emergency signs and to any subject patients without emergency signs and SpO2 < 90% (Clinical Management of COVID-19, Interim Guidance, May 27, 2020, WHO, WHO reference number: WHO/2019-nCoV/clinical/2020.5).

In an example, ICU may be required in cases of severe pneumonia, severe Acute Respiratory Distress Syndrome (ARDS), sepsis, and septic shock.

In an example, the prediction model can also perform a classification. On the basis of the subject patient data, at least one image of the lung is assigned to one of at least two classes, a first class and a second class. The first class comprises images showing the lung where ventilation and/or ICU is needed. The second class comprises images of the lung where not ventilation and/or ICU is need. In this case, the probability value represents the class the respective image is assigned to.

According to an example, the patient data further comprise at least one image of the aorta and/or heart of the subject patient suffering from COVID-19, and wherein the prediction model is trained by retrospective data to predict the relationship between visible features of COVID-19 in the lung in addition to visible features of COVID-19 in the aorta and/or heart and at least one characteristic of the COVID-19 disease progression of the subject patient suffering from COVID-19.

Similarly, as discussed herein above for images of the lung, the prediction model can be trained with a plurality of images of the aorta and/or heart of a plurality of patients that have suffered from COVID-19 and for which for each patient at least one characteristic of the COVID-19 progression has been determined in the past. The training is done to create a regression function which relates the visual features of the images of the lung, and the aorta and/or heart of a subject patient who has been infected with SARS-CoV-2 with the at least one characteristic of the COVID-19 progression. The extension to additional organs increases overall the accuracy of the prediction model.

According to an example, the visible features of COVID-19 in the lung comprise pulmonary abnormalities such as fibrosis and/or emphysema and wherein the visible features of COVID-19 in the heart and/or aorta comprise perfusion abnormalities.

According to an example, the patient data comprises at least two images of the heart and/or aorta for at least two different time points.

According to an example, the patient data comprises of at least one image selected from the group of a CT image, and an x-ray image.

In an example, the at least one image comprises a time stamp when the at least image has been acquired.

In an example, the patent data further comprises at least one bodily function data.

In an example, the artificial neuronal network has been trained with the additional (retrospective) bodily function data.

In an example, the bodily function data can by static and/or dynamic. Dynamic bodily function data can change over a short period of time (such as e.g. within hours or a day). Such dynamic bodily function data relates to data selected from the group of: heart rate, respiratory rate, body temperature, arterial oxygen saturation, fraction of inspired oxygen, Horowitz index, blood related data such as blood pH. Static body function data relates to data that does not change over time or data which changes very slowly. Such static body function data relates to data selected from the group of blood type, age, the presence of diseases such as cardiovascular disease, diabetes mellitus, chronic lung disease, cancer, and cerebrovascular disease. Recently, it has been published that certain blood types could potentially be linked to certain COVID-19 progressions (The ABO blood group locus and a chromosome 3 gene cluster associate with SARS-CoV-2 respiratory failure in an Italian-Spanish genome-wide association analysis; Ellinghaus et al., https://doi.org/10.1101/2020.05.31.20114991, doi: medRxiv preprint; June 2, 2020).

In an example, at least one bodily function data relates to dynamic data measured for at least two different time points. In an example, the measurement values of the same bodily function dynamic data are acquired at a plurality of different time points such as 10 measurement values within a time period of 12 hours.

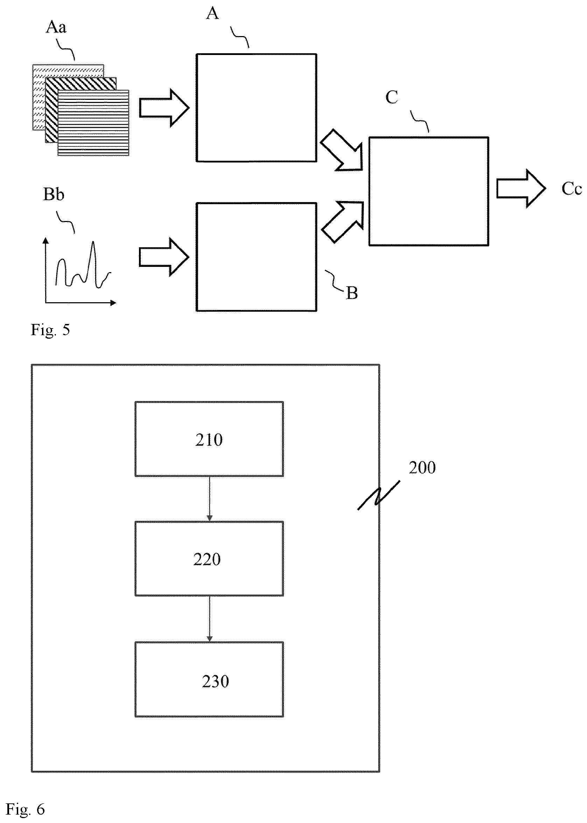

In an example, the at least one image of the lung, the at least one image of the heart and aorta and the at least one bodily function data are processed in the artificial neuronal network independently. The artificial neuronal network can have three subnetworks (see Fig. 5). The first subnetwork "A" serves to identify so-called image descriptors on the basis of the received patient data images (indicated in Fig. 5. with "Aa"). The second subnetwork "B" serves to identify so-called health descriptors on the basis of the at least one bodily function data (indicated in Fig. 5 with "Bb"). The first and the second subnetwork are merged into a third subnetwork "C". Thus, at least one layer of the first subnetwork and at least one layer of the second subnetwork is each connected with a layer of the third subnetwork. The third subnetwork can output the result(s) of the prediction model (indicated in Fig. 5 with "Cc").

In an example, the first subnetwork to generate the image descriptors is a Dense Convolutional Neural Network (DenseNet). Such networks are e.g. described in: G. Hunag et al.: Densely Connected Convolutional Networks, arXiv:1608.06993v5 [cs.CV] 28 Jan 2018.

In an example, the image descriptors and the health descriptors are time dependent descriptors. Time dependency in this context refers to the fact that the time stamp of when the data has been acquired is recorded and at least partially comprised in the descriptors or enclosed during the further processing through the artificial neuronal network.

In an example, the third subnetwork comprises a recurrent neuronal subnetwork (with backpropagation). Thus, the artificial neuronal network is able to consider the temporal development of the image data and the bodily function measurement data. As an example for a reccurent neuronal network a Long Short-Term Memory (LSTM) or a Time-Aware LSTM Network can be used (see e.g. I.M. Baytas et al.: Patient Subtyping via Time-Aware LSTM Networks, Proceedings of KDD '17, 2017, DOI: 10.1145/3097983.3097997).

In an example the first and/or second subnetwork comprise a recurrent neuronal subnetwork (with backpropagation).

In an example, the second subnetwork comprises an autoencoder and/or has been trained with the assistance of an autoencoder. The aim of an autoencoder is to learn a representation (encoding) for a set of data, typically for dimensionality reduction, by training the network to ignore signal "noise". Autoencoder are described e.g. in Q. V. Le: A Tutorial on Deep Learning Part 2: Autoencoders, Convolutional Neural Networks and Recurrent Neural Networks, 2015, https://cs.stanford.edu/~quocle/tutorial2.pdf; W. Meng: Relational Autoencoder for Feature Extraction, arXiv:1802.03145v1 [cs.LG] 9 Feb 2018; WO2018046412A1).

In an example, the second subnetwork comprises a reccurent neuronal network (with backpropagation) followed by an autoencoder.

According to an example, the method as described herein receives as an output of the prediction model at least one characteristic of the COVID-10 progression of a subject patient and its respective probability of occurrence.

In an example, the method receives as an output from the prediction model for each characteristic of a plurality of characteristics a probability for the occurrence of each characteristic.

In an example, the probability value can be outputted to a user and/or stored in a database such as the at least one characteristic of the COVID-19 progression as well. The probability value can be a real number in the range from 0 to 1, whereas a probability value of 0 means that there is no need for ventilation and/or ICU, and a probability value of 1 means that there is no doubt that there is a need for ventilation and/or ICU. In the context of the time until ventilation and/or ICU is needed, a time is indicated and the probability of 0 indicates that it is impossible that that time is applicable, wherein the probability of 1 indicates that it is very likely that the time is applicable. A similar interpretation is applicable to the length of stay with ventilation and/or length of stay with ICU. The probability value can also be expressed by a percentage.

A further embodiment of the invention relates to a computer-implemented method 200 for the determination of the treatment capacity for a plurality of subject patients suffering from COVID-19 in a hospital comprising:
- 210; analyzing at least one characteristic of a COVID-19 progression from a plurality of subject patients suffering from COVID-19 and hospitalized as determined with a method according to the first aspect of the invention,
- 220; determining the treatment capacity for a plurality of subject patients suffering from COVID-19 in the hospital as a function of the analysis of the at least one characteristic of the COVID-19 progression of the plurality of subject patients suffering from COVID-19,
- 230; receiving, as an output, the determination of the treatment capacity for a plurality of subject patients suffering from COVID-19 in the hospital.

Fig. 4 shows the method 200 schematically, in the form of a flow chart.

In an example, the treatment capacity can be determined by analyzing the at least one characteristic of the COVID-19 progression for each of the plurality of subject patients.

In an example, "analyzing" refers to first the determination whether ventilation and/or ICU for a subject patient is needed. Second, if ventilation and/or ICU is needed, it refers to the time until ventilation and/or ICU is needed and third, it refers to the length of stay with ventilation, in an ICU and/or in the hospital.

According to an example, the at least one characteristic of a COVID-19 progression comprises at least one of the following:
- Need for ventilation of the plurality of subject patients suffering from COVID-19,
- Time until the plurality of subject patients suffering from COVID-19 needs ventilation if need for ventilation has been determined,
- Length of stay of the plurality of subject patients suffering from COVID-19 with ventilation if a need for ventilation has been determined,
- Need for the plurality of subject patients suffering from COVID-19 for intensive care in an ICU of the hospital,
- Time until the plurality of subject patients suffering from COVID-19 need intensive care in an ICU of the hospital,
- Length of stay of the plurality of subject patients suffering from COVID-19 in the ICU of the hospital,
- Length of stay of the plurality of subject patients suffering from COVID-19 in the hospital.

According to an example, the determination of the treatment capacity for a plurality of subject patients suffering from COVID-19 in a hospital as a function of the analysis of the at least one characteristic of the COVID-19 progression of the plurality of subject patients suffering from COVID-19 comprises at least one of the following:
- allocation of available ventilation devices in the hospital to a plurality of subject patients suffering from COVID-19 according to the determined characteristics of the COVID-19 progression for each subject patient,
- allocation of ICUs in the hospital to a plurality of subject patients suffering from COVID-19 according to the determined characteristics of the COVID-19 progression for each subject patient.

Therefore, in an example the determination of the treatment capacity in the hospital refers to the allocation of the ventilation devices that are available in the hospital and/or the the ICUs that are available in the hospital to the COVID-19 patients that require ventilation and/or intensive care.

In an example, the "allocation of ventilation devices in the hospital" refers to when and by whom (subject patient suffering from COVID-19) a ventilation device is used in the hospital.

In an example, the "allocation of ICUs in the hospital" refers to when and by whom (subject patient suffering from COVID-19) an ICU is used in the hospital.

In an example, the allocation time period is determined for a time period of e.g. 1 to 14 days, preferably for a time period of 1 to 7 days, more preferably 1 to 3 days and can be updated e.g. daily or hourly be re-running the computer-implemented methods as described herein and including additional patient data of subject patients for which a determination of the at least one characteristic of the COVID-19 progression has already being made and/or by including patient data of subject patients that have in the mean time being hospitalized or that have already being hospitalized and in the mean time being tested positive for SARS-CoV-2. In an example, the probability for the occurrence of each characteristic of the COVID-19 progression of the plurality of subject patients is considered when the treatment capacity is determined. For example, if the probability for the need of ventilation for a subject patient is determined to be below a predetermined threshold value (e.g. such as 5%) no ventilation device is allocated to such a subject patient.

In an example, the term "ventilation device" refers to any type of device suitable for the ventilation of a subject patient and to be partly positioned inside the trachea of said patient. Said ventilation device may for instance refer to an endotracheal or tracheostomy tube. In general, said ventilation device refers to a flexible plastic catheter to be placed into the trachea of a patient to protect the airway and provide a means of mechanical ventilation.

According to an example, the determination of the treatment capacity for a plurality of subject patients suffering from COVID-19 in a hospital as a function of the analysis of the at least one characteristic of the COVID-10 progression of the plurality of subject patients suffering from COVID-19 comprises at least one of the following:
- Request of ventilation devices from a third party if the need for ventilation devices has been predicted to succeed the availability of ventilation devices in the hospital,
- Transport of the required ventilation devices from the third party to the hospital,
- Request the hospitalization of (an) eligible subject patient(s) suffering from COVID-19 in another hospital with available ICUs if the need for ICUs in the hospital has been predicted to exceed the availability of ICUs in the hospital,
- Transfer of (a) eligible subject patient(s) suffering from COVID-19 to another hospital with available ICUs.

In an example, the "determination of the treatment capacity" in the method described above extends to the "management of the treatment capacity".

In an example, first, the allocation of the ventilation devices and the ICUs that are available within a certain time period (the allocation time period as discussed above) in the hospital are determined. If the demand for ventilation devices and/or ICUs exceed the availability for at least an hour (or at least 5 hours, or at least one day) within the allocation time period in the hospital, ventilation devices can be requested from third parties and/or eligible subject patients suffering from COVID-19 can be transferred to another hospital if they are likely to be in need for ICU in the near future.

In an example, a third party in the context of the invention can be a company, institution or any other legal entity which can offer, sell, and/or loan ventilation devices.

In an example, an eligible subject patient suffering from COVID-19 for the transfer to an ICU of another hospital is a patient that is determined to need ICU, but not immediately.

Another embodiment of the invention relates to a system 300 comprising:
- a patient data receiving unit 310 for receiving patient data of a subject patient who has been infected with SARS-CoV-2, the patient data comprising at least one image of the lung of the subject patient;
- a memory 320;
- a processing unit 330 in communication with the memory and configured with processor-executable instructions to perform operations comprising:
- feeding the patient data of the subject patient into a prediction model, wherein the prediction model is trained by retrospective data to predict the relationship between visible features of COVID-19 in the lung and at least one characteristic of the COVID-19 progression of the subject patient suffering from COVID-19,
- receiving as an output from the prediction model the at least one characteristic of the COVID-19 progression of the subject patient suffering from COVID-19,
- outputting the at least one characteristic of the COVID-19 progressoin of the subject patient by an output unit 340.

In an example, the system 300 implements the method 100. Fig. 5 shows schematically the system 300 which is in an example a computer system. Generally, a computer system of exemplary implementations of the present disclosure may be referred to as a computer and may comprise, include, or be embodied in one or more fixed or portable electronic devices. The computer may include one or more of each of a number of components such as, for example, patient data receiving unit 310, a processing unit 330 connected to a memory 320 (e.g., storage device), an output unit 340. The processing unit 330 may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory 320 of the same or another computer. The processing unit 330 may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure. The memory 320 is generally any piece of computer hardware that is capable of storing information such as, for example, data, computer programs (e.g., computer-readable program code) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium. In addition to the memory 320, the processing unit 330 may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) to connect to a network, such as using technologies such as cellular telephone, Wi-Fi, satellite, cable, digital subscriber line (DSL), fiber optics and the like. In some examples, the communications interface(s) may include one or more short-range communications interfaces configured to connect devices using short-range communications technologies such as NFC, RFID, Bluetooth, Bluetooth LE, ZigBee, infrared (e.g., IrDA) or the like. The user interfaces may include a display. The display may be configured to present or otherwise display information to a user, suitable examples of which include a liquid crystal display (LCD), light-emitting diode display (LED), plasma display panel (PDP) or the like. The user input interface(s) may be wired or wireless, and may be configured to receive information from a user into the system, such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

Another aspect of the invention relates to a system 400 comprising:
- a patient data receiving unit 410 for receiving patient data of a plurality of subject patients who have been infected with SARS-CoV-2, the patient data comprising at least one image of the lung of the plurality of subject patients;
- a memory 420;
- a processing unit 430 in communication with the memory and configured with processor-executable instructions to perform operations comprising:
- feeding the plurality of patient data from a plurality of subject patients into a prediction model, wherein the prediction model is trained by retrospective data to predict the relationship between visible features of COVID-19 in the lung and at least one characteristic of the COVID-19 progression of a subject patient suffering from COVID-19,
- receiving as an output from the prediction model at least one characteristic of the COVID-19 progression of a plurality of subject patients suffering from COVID-19,
- analyzing at least one characteristic of the COVID-19 progression of the plurality of subject patients suffering from COVID-19,
- determining the treatment capacity for a plurality of subject patients suffering from COVID-19 in a hospital as a function of the comparison of the at least one characteristic of the COVID-19 progression of the plurality of subject patients suffering from COVID-19,
- outputting the results of the determination of the treatment capacity in a hospital for a plurality of subject patients suffering from COVID-19 progression by an output unit 440.

In an example, the system 400 implements the method 300. Fig. 6 shows schematically the system 400.

In an example, the determination of the at least one characteristic of the COVID-19 progression of a subject patient is similar as described in the context of the method 100. In an example, the system 400 comprises similar computer system components as described in the context of the system 300.

A further embodiment of the invention relates to a non-transitory computer-readable storage medium comprising processor-executable instructions with which to perform an operation for determining the impact of the COVID-19 progression of a subject patient suffering from COVID-19 on a hospital and its ICUs, the operation comprising:
- receiving patient data of a subject patient who has been infected with SARS-CoV-2, the patient data comprising at least one image of the lung of the subject patient,
- inputting the patient data into a prediction model trained via machine learning with a set of training data, wherein the prediction model is trained by retrospective data to predict the relationship between visible features of COVID-19 in the lung and at least one characteristic of the COVID-19 progression of the subject patient suffering from COVID-19,
- receiving as an output from the prediction model the at least one characteristic of the COVID-19 progression of the subject patient suffering from COVID-19.

In an example, the above described non-transitory computer readable storage medium comprises the information to perform the method 100.

Another embodiment of the invention relates to a non-transitory computer-readable storage medium comprising processor-executable instructions with which to perform an operation for determining the treatment capacity for a plurality of subject patients suffering from COVID-19 in a hospital, the operation comprising:
- analyzing at least one characteristic of the of a COVID-19 progression from a plurality of subject patients suffering from COVID-19 and hospitalized as determined with the operations as described for the non-transitory computer-readable storage medium discussed above,
- determining the treatment capacity for a plurality of subject patients suffering from COVID-19 in the hospital as a function of the comparison of the at least one characteristic of the COVID-19 progression of the plurality of subject patients suffering from COVID-19,
- receiving as an output the results of the determination of the treatment capacity for a plurality of subject patients suffering from COVID-19 in the hospital.

In an example, the above described non-transitory computer readable storage medium comprises the information to perform the method 200.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g. digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g. electronic, phenomena which may occur or reside e.g. within registers and/or memories of at least one computer or processor. The term processor includes a single processing unit or a plurality of distributed or remote such units.

Any suitable processor/s, display and input means may be used to process, display e.g. on a computer screen or other computer output device, store, and accept information such as information used by or generated by any of the methods and system shown and described herein; the above processor/s, display and input means including computer programs, in accordance with some or all of the embodiments of the present invention. Any or all functionalities of the invention shown and described herein, such as but not limited to operations within flowcharts, may be performed by any one or more of: at least one conventional personal computer processor, workstation or other programmable device or computer or electronic computing device or processor, either general-purpose or specifically constructed, used for processing; a computer display screen and/or printer and/or speaker for displaying; machine-readable memory such as optical disks, CDROMs, DVDs, BluRays, magnetic-optical discs or other discs; RAMs, ROMs, EPROMs, EEPROMs, magnetic or optical or other cards, for storing, and keyboard or mouse for accepting. Modules shown and described herein may include any one or combination or plurality of: a server, a data processor, a memory / computer storage, a communication interface, a computer program stored in memory/computer storage. Any suitable input device, such as but not limited to a camera sensor, may be used to generate or otherwise provide information received by the system and methods shown and described herein. Any suitable output device or display may be used to display or output information generated by the system and methods shown and described herein. Any suitable processor/s may be employed to compute or generate information as described herein and/or to perform functionalities described herein and/or to implement any engine, interface or other system described herein. Any suitable computerized data storage e.g. computer memory may be used to store information received by or generated by the systems shown and described herein. Functionalities shown and described herein may be divided between a server computer and a plurality of client computers. These or any other computerized components shown and described herein may communicate between themselves via a suitable computer network.

Some implementations of the present disclosure are described with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art. As used herein, for example, the singular forms "a", "an", "the" and the like include plural referents unless the context clearly dictates otherwise. The terms "data", "information", "content" and similar terms may be used interchangeably, according to some example implementations of the present invention, to refer to data capable of being transmitted, received, operated on, and/or stored. Also, for example, reference may be made herein to quantitative measures, values, relationships or the like. Unless otherwise stated, any one or more if not all of these may be absolute or approximate to account for acceptable variations that may occur, such as those due to engineering tolerances or the like. Like reference numerals refer to like elements throughout.

## Claims

1. A computer-implemented method for determining the impact of COVID-19 progression of a subject patient suffering from COVID-19 on a hospital and its Intensive Care Unit (ICU), comprising:
- receiving patient data of a subject patient who has been infected with SARS-CoV-2 and hospitalized, wherein the patient data comprises at least one image of the lung of the subject patient,
- inputting the patient data into a prediction model trained via machine learning with a set of training data, wherein the prediction model is trained by retrospective data to predict the relationship between visible features of COVID-19 in the lung and at least one characteristic of the COVID-19 progression of the subject patient suffering from COVID-19,
- receiving, as an output from the prediction model, at least one characteristic of the COVID-19 disease progression of the subject patient suffering from COVID-19.

2. Method according to claim 1, wherein the at least one at least one characteristic of the COVID-19 progression is selected from the group of:
- Need for ventilation of the subject patient suffering from COVID-19,
- Time until subject patient suffering from COVID-19 needs ventilation if a need for ventilation has been determined,
- Length of stay of the subject patient suffering from COVID-19 with ventilation if a need for ventilation has been determined,
- Need for the subject patient suffering from COVID-19 for intensive care in an ICU of a hospital,
- Time until subject patient suffering from COVID-19 needs intensive care in an ICU of a hospital,
- Length of stay of the subject patient suffering from COVID-19 in the ICU of a hospital,
- Length of stay of the subject patient suffering from COVID-19 in the hospital.

3. Method according any one of claims 1 to 2, wherein the patient data further comprise at least one image of the aorta and/or heart of the subject patient suffering from COVID-19, and wherein the prediction model is trained by retrospective data to predict the relationship between visible features of COVID-19 in the lung in addition to visible features of COVID-19 in the aorta and/or heart and at least one characteristic of the COVID-19 disease progression of the subject patient suffering from COVID-19.

4. Method according to claim 3, wherein the visible features of COVID-19 in the lung comprise pulmonary abnormalities such as fibrosis and/or emphysema and wherein the visible features of COVID-19 in the heart and/or aorta comprise perfusion abnormalities.

5. Method according to claim 4, wherein the patient data comprises at least two images of the heart and/or aorta for at least two different time points.

6. Method according to any one of the previous claims, wherein the patient data comprises at least one image selected from the group of a CT image, and an x-ray image.

7. Method according to any one of the previous claims, wherein an output of the prediction model is at least one characteristic of the COVID-10 progression of a subject patient and its respective probability of occurrence.

8. A computer-implemented method for the determination of the treatment capacity for a plurality of subject patients suffering from COVID-19 in a hospital comprising:
- analyzing at least one characteristic of a COVID-19 progression from a plurality of subject patients suffering from COVID-19 and hospitalized as determined with a method according to any one of the claims 1 to 7,
- determining the treatment capacity for a plurality of subject patients suffering from COVID-19 in the hospital as a function of the analysis of the at least one characteristic of the COVID-19 progression of the plurality of subject patients suffering from COVID-19,
- receiving, as an output, the determination of the treatment capacity for a plurality of subject patients suffering from COVID-19 in the hospital.

9. Method according to claim 8, wherein the at least one characteristic of a COVID-19 progression comprises at least one of the following:
- Need for ventilation of the plurality of subject patients suffering from COVID-19,
- Time until the plurality of subject patients suffering from COVID-19 needs ventilation if need for ventilation has been determined,
- Length of stay of the plurality of subject patients suffering from COVID-19 with ventilation if a need for ventilation has been determined,
- Need for the plurality of subject patients suffering from COVID-19 for intensive care in an ICU of the hospital,
- Time until the plurality of subject patients suffering from COVID-19 need intensive care in an ICU of the hospital,
- Length of stay of the plurality of subject patients suffering from COVID-19 in the ICU of the hospital,
- Length of stay of the plurality of subject patients suffering from COVID-19 in the hospital.

10. Method according to any of claim 8 or 9, wherein the determination of the treatment capacity for a plurality of subject patients suffering from COVID-19 in a hospital as a function of the analysis of the at least one characteristic of the COVID-19 progression of the plurality of subject patients suffering from COVID-19 comprises at least one of the following:
- allocation of available ventilation devices in the hospital to a plurality of subject patients suffering from COVID-19 according to the determined characteristics of the COVID-19 progression for each subject patient,
- allocation of ICUs in the hospital to a plurality of subject patients suffering from COVID-19 according to the determined characteristics of the COVID-19 progression for each subject patient.

11. Method according to any of claim 10, wherein the treatment capacity for a plurality of subject patients suffering from COVID-19 in a hospital as a function of the analysis of the at least one characteristic of the COVID-10 progression of the plurality of subject patients suffering from COVID-19 comprises at least one of the following:
- Request of ventilation devices from a third party if the need for ventilation devices has been predicted to succeed the availability of ventilation devices in the hospital,
- Transport of the required ventilation devices from the third party to the hospital,
- Request the hospitalization of (an) eligible subject patient(s) suffering from COVID-19 in another hospital with available ICUs if the need for ICUs in the hospital has been predicted to exceed the availability of ICUs in the hospital,
- Transfer of (a) eligible subject patient(s) suffering from COVID-19 to another hospital with available ICUs.

12. A system comprising:
- a patient data receiving unit for receiving patient data of a subject patient who has been infected with SARS-CoV-2, the patient data comprising at least one image of the lung of the subject patient;
- a memory;
- a processing unit in communication with the memory and configured with processor-executable instructions to perform operations comprising:
- feeding the patient data of the subject patient into a prediction model, wherein the prediction model is trained by retrospective data to predict the relationship between visible features of COVID-19 in the lung and at least one characteristic of the COVID-19 progression of the subject patient suffering from COVID-19,
- receiving as an output from the prediction model the at least one characteristic of the COVID-19 progression of the subject patient suffering from COVID-19,
- outputting the at least one characteristic of the COVID-19 progressoin of the subject patient by an output unit.

13. A system comprising:
- a patient data receiving unit for receiving patient data of a plurality of subject patients who have been infected with SARS-CoV-2, the patient data comprising at least one image of the lung of the plurality of subject patients;
- a memory;
- a processing unit in communication with the memory and configured with processor-executable instructions to perform operations comprising:
- feeding the plurality of patient data from a plurality of subject patients into a prediction model, wherein the prediction model is trained by retrospective data to predict the relationship between visible features of COVID-19 in the lung and at least one characteristic of the COVID-19 progression of a subject patient suffering from COVID-19,
- receiving as an output from the prediction model at least one characteristic of the COVID-19 progression of a plurality of subject patients suffering from COVID-19,
- analyzing at least one characteristic of the COVID-19 progression of the plurality of subject patients suffering from COVID-19,
- determining the treatment capacity for a plurality of subject patients suffering from COVID-19 in a hospital as a function of the comparison of the at least one characteristic of the COVID-19 progression of the plurality of subject patients suffering from COVID-19,
- outputting the results of the determination of the treatment capacity in a hospital for a plurality of subject patients suffering from COVID-19 progression by an output unit.

14. A non-transitory computer-readable storage medium comprising processor-executable instructions with which to perform an operation for determining the impact of the COVID-19 progression of a subject patient suffering from COVID-19 on a hospital and its ICUs, the operation comprising:
- receiving patient data of a subject patient who has been infected with SARS-CoV-2, the patient data comprising at least one image of the lung of the subject patient,
- inputting the patient data into a prediction model trained via machine learning with a set of training data, wherein the prediction model is trained by retrospective data to predict the relationship between visible features of COVID-19 in the lung and at least one characteristic of the COVID-19 progression of the subject patient suffering from COVID-19,
- receiving as an output from the prediction model the at least one characteristic of the COVID-19 progression of the subject patient suffering from COVID-19.

15. A non-transitory computer-readable storage medium comprising processor-executable instructions with which to perform an operation for determining the treatment capacity for a plurality of subject patients suffering from COVID-19 in a hospital, the operation comprising:
- analyzing at least one characteristic of the of a COVID-19 progression from a plurality of subject patients suffering from COVID-19 and
hospitalized as determined with the operations as described according to the fifth aspect of the invention,
- determining the treatment capacity for a plurality of subject patients suffering from COVID-19 in the hospital as a function of the comparison of the at least one characteristic of the COVID-19 progression of the plurality of subject patients suffering from COVID-19,
- receiving as an output the results of the determination of the treatment capacity for a plurality of subject patients suffering from COVID-19 in the hospital.
